# EUROPEAN PATENT APPLICATION

(11) **EP 1 252 826 A1**
(43) Date of publication of application: **30.10.2002**
(21) Application number: 01201533.5
(22) Date of filing: 26.04.2001
(51) Int. Cl.: A23L 1/236, C07K 5/06

(54) **Non-caking aspartame fine grade**

(71) Applicant: Holland Sweetener Company V.o.F., 6167 RA Geleen (NL)
(72) Inventor: Kuehn, Frank Thomas, 6163 KC Geleen (NL); Damen, Vera Henrica Johanna Thomas, 6229 GD Maastricht (NL)

(57) **Abstract**

The invention relates to a method for producing an improved fine grade of α-L-aspartyl-L-phenylalanine methyl ester (aspartame; APM), having a particle size smaller than 100 µm, which has been produced by crystallisation under agitated conditions, granulation and subsequent mechanical size reduction, wherein the mechanical size reduction of the APM is carried out so as to obtain a first product of particles, smaller than 100 µm, from which in a further step the fraction of finest particles is removed to a large extent by dry classifying and APM is obtained having a d₁₀ value of at least 3 µm and a d₅₀ value in the range of from 15 to 65 µm, the d-values as determined by laser diffraction particle size measurement. In particular the invention relates to producing an improved fine grade of APM which may be handled easily and can suitably be used in powder mixes and other applications, such as chewing gum and tableting. The invention also relates to such improved novel fine grade products of APM having a d₁₀ value of at least 3 µm, a d₅₀ value in the range of from 15 to 65 µm, and a free bulk density of at least 270 kg/m³. The particle size distribution of such novel APM products, when represented graphically in a so-called Rosin-Rammler-Sperling-Bennett (RRSB) grid, shows a substantially steeper slope in the range between d₂₅ and d₉₀ than in the range between d₁₀ and d₂₀.

## Description

The invention relates to a method for producing an improved fine grade of α-L-aspartyl-L-phenylalanine methyl ester (hereinafter also referred to as aspartame, or as APM), having a particle size that is in the main smaller than 100 µm. As meant herein, APM means any product consisting entirely of
α-L-aspartyl-L-phenylalanine methyl ester, including however such minor quantities of other products co-crystallised therewith and/or present in the water in or freely adhering to the dried APM as obtained in its production process. The amount of water (crystal water and free water) in the (dried) APM, as meant herein, usually will not exceed about 4% by weight (i.e. ≤ 4 wt.%). The term "in the main" as used herein indicates that at least 99% by weight of the particles, as for instance can be determined by laboratory air-jet sieve tests or by laser-diffraction methods, has a size smaller than 100 µm. More particularly the invention relates to producing an improved fine grade of APM which may be handled easily and can suitably be used in powder mixes, from APM which has been produced by crystallisation under agitated conditions, granulation and subsequent mechanical size reduction of the particles formed. The invention also relates to such improved fine grade products and the use thereof for sweetening edible products.

α-L-Aspartyl-L-phenylalanine methyl ester (aspartame; APM) is a dipeptide sweetener with a sweetening power that is about 200x that of sugar. Aspartame is widely used as a sweetener in a wide diversity of edible products, soft drinks, confectionery, medicines and in table sweeteners and the like. APM is often used in the form of dry blends, such as instant powdered drinks and instant dessert products and the like.

Fine grade APM, obtained through crystallisation under agitated conditions and having a particle size that is in the main smaller than 100 µm, is known from EP-A-0800774, where such products are being used for being deposited on (i.e. for coating of) an edible supporting material. In said reference various examples are shown, referred to as products "A3a." to "A3g.". Those products have been characterised by (i) values of moisture content (LOD; loss on drying measured as percentage of weight loss after 4 hours heating in an open dish at 105°C) in the range of 2.6 to 4.5%; (ii) particle size distribution (p.s.d.) as estimated by microscopic evaluation as percentage by weight of the particles < 80 µm, respectively < 50 µm and < 20 µm; and by their free bulk density (f.b.d.; determined according to ASTM D1895-89 (1990)) in the range of from 161 to 289 kg/m³. These products were all obtained by size reduction with simultaneous classification of particles above the upper particle size limit.

Instead of mentioning weight percentages of particles below a certain value of particle size, as above (and as done in the cited reference), one also might use characterisation of particle size distribution by means of so-called dₓₓ values for a given particle size, xx representing any figure between 0 and 100.

A d₅₀ value of, for example, 100 µm is in the context of this application understood to mean that 50 wt.% of the particles of the product concerned (powder) have a particle size such that this amount is retained on a sieve with an aperture size of 100 µm. The values for the d₁₀, etc., mentioned in this application should be interpreted analogously, although it will be understood, that for particle sizes of less than 50 µm such sieving method in general is less suitable. Instead of assessing the dₓₓ values by means of sieving tests, however, especially for particle sizes below 100 µm, other methods are more appropriate, for instance laser-diffraction methods, Coulter counter (using electric field disturbance), time-of-flight measurement (e.g. using an Aerosizer of Amhurst Process Instruments), Photon Correlation Spectroscopy, etc.

Although these products as disclosed in EP-A-0800774 have very favourable properties for being applied as a coating to edible carrier materials of appropriate particle size distribution, they have a tendency to exhibit strong caking when being stored and, to the extent they do not form a layer on the carrier materials, to form more or less spherical and well-visible agglomerates of APM when being mixed with other components. The latter phenomenon might be called "balling", although this term is not being used in literature. In other words, the caking is the tendency of formation of relatively hard lumps of APM due to time-consolidation: for APM residence time, pressure and migration of moisture appear to play an important role in its caking behaviour. Balling, on the other hand, is the tendency of formation - during mixing with other materials - of agglomerates, which are difficult to disintegrate and are composed of particles of APM. As a result this leads to undesirable visibility of at least some white APM-agglomerates in the mixes obtained, especially in mixtures with coloured materials. Generally, the human eye is able to detect particles of about 100 µm or larger without assistance of microscopic techniques, particularly under high contrast conditions of particles versus their background.

Moreover, the mixing behaviour of these APM products when being mixed together with various other components, such as maltodextrins, citric acid, fructans, cyclodextrins, sugar alcohols, carbohydrates, cocoa, etc. is often insufficient because inhomogeneous mixtures are being obtained, especially when mixing is done at too low mixing energy input to break-up the agglomerates.

On the other hand, APM powders, consisting entirely of APM, for application in instant powder mixtures etc., are known from EP-A-0915667. As disclosed therein, these APM powders can be obtained from dry or wet APM granules by first treating them - while air is being supplied for discharging the crushed product - in an impact mill, which optionally is provided with a coarse sifter to separate oversize particles out of the air stream. In said first step a product is obtained in which at least 70 wt.% of the particles is smaller than 150 µm. The fraction of particles < 20 µm in the crushed product so obtained is then reduced to < 10 wt.% with the aid of a fines sifter without separating any product from the air discharged from the impact mill before leading said discharge stream through the fines sifter. Said method allows to obtain APM powders of rather narrow particle size distribution, with a d₅₀ value in the range of 40 to 80 µm. It is taught that the amount of the size fraction < 20 µm preferably should be at least 3 wt.% and no more than 5 wt.%. The APM powders so-obtained, however have a relatively strong tendency to segregate, thereby becoming inhomogeneous and less consistent in product properties. Of course, this also negatively influences the mixing behaviour of such APM products.

Unfortunately, the use of aspartame (powder) entirely or practically entirely consisting of aspartame as a starting material for the preparation of powder mixtures indeed hitherto often poses problems in the practical preparation of such mixtures. In the context of the present application, entirely or practically entirely consisting of aspartame means that the product in question contains no other components than the amounts of impurities and moisture present and falling within the normal product specifications for APM. Hereinafter, this is also referred to as 100 wt.% APM. None of the commercially available aspartame products (powders and granular products based on 100 wt.% APM) known to date are particularly suitable for all such applications in powder mixtures (nor in other applications, such as use in chewing gum or in tableting) over the whole range thereof.

The use of APM, in particular of powdered or fine-grade APM, is thus in general often hampered in terms of handling by the fact that the products in question show poor flow behaviour, but also to a large extent may be correlated to the caking behaviour of the APM fine grade products. In addition, also importantly, the balling behaviour of the APM fine grade products needs to be at an acceptably low level, and visibility of particles in powder mixtures, especially with dark coloured products, such as cocoa, should be avoided.

According to generally known principles, the caking behaviour of powdered products can be expressed in terms of changes in values of flowability over time, e.g. when the product is stored under defined conditions for 2 weeks. A suitable way of quantifying flowability of a product is by means of its *ff*_{*c*} value, a value introduced by Jenike (see J. Schwedes, in Powder Handling & Processing, Vol.12(4), October/December 2000, pages 337-354, especially at page 341). The *ff*_{*c*} value for a product is the ratio of a product's major principal stress at steady state flow (σ₁) to the so-called unconfined yield strength (σ_{c}), both of which sigma-values can be determined by using a Jenike shear tester, consisting of a base (A), a ring (B) resting on top of the base and a lid (C). (For procedures of testing, etc. see the reference cited). Tomas et al. (Partec 79, Particle characterisation, Nürnberg, Germany, 1979, p. 301-319) have presented an extended classification of products according to their *ff*_{*c*} values, as follows:

| | |
|---|---|
| *ff*_{*c*} value < 1 | hardened |
| *1* < *ff*_{*c*} value < 2 | very cohesive |
| 2 < *ff*_{*c*} value < 4 | cohesive |
| 4 < *ff*_{*c*} value < 10 | easy flowing |
| 10 < *ff*_{*c*} value | free flowing. |

For the APM products according to the state of the art according to EP-A-0915667 and EP-A-0800774 *ff*_{*c*} values are being found of about 1,5 to 1,8 immediately after their production, respectively of 0,6 after 2 weeks of storage under a pressure of 3275 N/m². It is noticed that such products have been obtained by crystallisation under agitated conditions. These *ff*_{*c*} values, especially those after a sufficiently long period, for instance after 2 weeks of storage under a certain pressure (that after such period giving a good indication of the caking behaviour), are considered to be quite inadequate for such products being used in powder mixes, chewing gums, tableting and other applications, unless complete coating of carrier material particles is to be achieved under proper energy input conditions. It is, moreover, to be noticed that caking, contrary to agglomeration, is a rather irreversible process, and thus should be avoided.

There is hence a need for a simple and efficient method for providing a homogeneous APM product having a particle size that is in the main smaller than 100 µm from α-L-aspartyl-L-phenylalanine methyl ester which has been produced by crystallisation under agitated conditions, granulation and subsequent mechanical size reduction of the particles formed with adequate dissolution and improved handling properties, in particular a low tendency of caking on storage and of balling during mixing, and sufficiently high free bulk density.

Surprisingly it now has been found, that an improved fine grade of α-L-aspartyl-L-phenylalanine methyl ester having a particle size that is in the main smaller than 100 µm can be produced from α-L-aspartyl-L-phenylalanine methyl ester which has been produced by crystallisation under agitated conditions, granulation and subsequent mechanical size reduction of the particles formed, if the mechanical size reduction of α-L-aspartyl-L-phenylalanine methyl ester is carried out so as to obtain in the mechanical size reduction step a first product consisting of particles, which in the main are smaller than 100 µm, from which in a further step the fraction of finest particles is removed to a large extent by dry classifying and α-L-aspartyl-L-phenylalanine methyl ester is obtained having a d₁₀ value of at least 3 µm and a d₅₀ value in the range of from 15 to 65 µm, the d-values as determined by laser diffraction particle size measurement.

In this method of production:
# "α-L-Aspartyl-L-phenylalanine methyl ester (APM), having a particle size that is in the main smaller than 100 µm" means any product consisting entirely of α-L-aspartyl-L-phenylalanine methyl ester, such low quantities of other products co-crystallised therewith and/or present in the water in or freely adhering to the dried APM as obtained in its production process. The amount of water (crystal water and free water) in the (dried) APM, as meant herein, usually will not exceed about 4% by weight (i.e. ≤ 4 wt.%).
# "Particles" means primary particles; agglomerated particles are being referred to herein as "agglomerates".
# "A particle size that is in the main smaller than 100 µm" has the meaning as described in the first paragraph hereof.
# "Crystallisation under agitated conditions" means that crystallisation takes place under forced convection; this leads to crystals which are significantly different from those obtained by so-called static crystallisation, i.e. under conditions without forced convection.
# "Granulation and subsequent mechanical size reduction of the particles" means that the crystallised APM is granulated and dried, for instance in a subsequent steps or simultaneously, for instance using a high-speed paddle dryer, employing such conditions that the amount of water of the (dried) APM so-obtained does not exceed 4 wt.%; in the subsequent mechanical size reduction, all kinds of mechanical size reduction methods known to the skilled man can be used, for instance by grinding, or milling, or crushing.
# "Classifying" means any possible classification technique for controlling the particle size distribution of a product by removing, to a large extent (i.e. complete removal will be virtually impossible, nor necessary) as is controlled, a fraction (for instance of the finest or largest particles present in a product) from such product.
   Dry classification of particles < 50 µm (in particular < 20 µm) according to the method of the invention can be done by any known technique of dry classification. Preferably use is made of air-classification techniques, especially in case the mechanical size reduction of the particles is being done while using drying air in an impact mill, for instance as is being taught in EP-A-0915667. Instead of air, of course, also inert gases may be used for assisting the classification. The air, or such other inert gas, preferably has a moisture content controlled or conditioned in such way, that the amount of water in the final product produced according to the invention will not exceed 4 wt.%, and preferably will be lower than 2,5 wt.%. Tendency to form lumps will be lower as the amount of water in the product is lower.
   Such air classification may be performed (a) in cross-flow configuration, or (b) by counter flow, each of which methods may be combined with either (i) a gravity field, or (ii) a centrifugal field. In the process according to the invention counter flow air classification in a centrifugal field, such as, for instance, can be achieved in a so-called deflector wheel classifier, is most preferred. For instance, cyclone classifiers may be used, as are provided by Hosokawa Micron GmbH or Neuman & Esser GmbH (Übach-Palemberg, Germany). In such cyclone classifiers the deflector wheel is inserted into a cyclone geometry housing. Advantage of this design is that a secondary air flow can be applied so that fine particles in the classified product stream are blown back into the classification zone of the cyclone to improve the sharpness of cut and to improve the cut-size.
   Alternatively, said classification may be done using an air-jet sieve, for instance one supplied by Airtechnic B.V. (Venray, the Netherlands; APPI classifier), or by Sweco.
# "d₁₀ and d₅₀ values", as defined by a maximum value of the crystal size, or by a range of values of the crystal size, in µm, have the meaning as given hereinabove in general terms for dₓₓ values; in particular these values are the values as determined by by laser diffraction particle size measurement. For details, please see the experimental part.

The process according to the present invention ensures that the problems to be solved are being overcome properly. As an additional advantage it has been found, that the products so-obtained are excellently suitable for being used in tableting, especially using wet granulation techniques or even in tableting by direct compression. In this context it is to be noticed, that α-L-aspartyl-L-phenylalanine methyl ester generally is known to behave differently from most other organic compounds.

The particle size of the APM obtained after crystallisation, separation, drying, granulation and size reduction, which is produced in the size reduction step as a first product in the method according to the present invention, is such that at least 99 wt.% of the particles is < 100 µm.

It is to be noticed here, that the skilled man, at the date of the invention would not have expected removal of particles < 20 µm from APM having a particle size of 100 µm or less to be a suitable technique. In the past, for instance, it has also been attempted to prepare suitable aspartame powders according to a method as described in EP-A-0574983. In said reference it is described that aspartame containing at least 5 wt.% of particles < 20 µm (hereinafter also referred to as dust or fines) and at least 10 wt.% of particles > 400 µm is subjected to a multistage fractionation process: in a first step, a large proportion of the "small" particles (i.e. particles < 50, or < 40, or < 30, or < 20 µm) is removed by treatment in a fluid bed or with, for example, a Sweco Turbo Screen; subsequently, in a second step, a so-called initial product is obtained by sieving out the particles that are larger than a given upper limit in the range from 150 to 250 µm.

The particle size distribution of the products made according to the processes mentioned in EP-A-0574983 will generally be such that the products have a d₅₀ in the range of from about 80 to about 130 µm and a d₉₇ in the range of from about 150 to about 250 µm. It appears not to be possible, using the method according to said patent publication, to obtain a product which has a d₉₇ < 150 µm and at the same time contains less than 3 wt.% < 20 µm.

Moreover, as is disclosed in EP-A-0915667, it has in the meantime been found by applicant that the technique of EP-A-0574983 operates satisfactorily only if the dust content (i.e. particles < 20 µm) of the starting material to be processed is not higher than approx. 20-30 wt.%. When that same technique is applied in, for example, a fluid bed with such dust contents of 20-30 wt.% or higher, fines removal, or dedusting, appears to be very difficult because either the starting product fails to fluidise in the fluid bed. Moreover, a sharp cut in particle sizes is impossible when using dedusting techniques, so that too large amounts would be blown-out of product having particle sizes larger than the ones to be removed. Dedusting does not occur even when attempts are made to fluidise the fluid bed by mechanical means (for example by vibration or application of a stirrer). Nor is a suitable technique as needed in the framework of the present invention obtained when an APM starting material with such high dust contents is treated in a Sweco Turbo Screen; plugging problems often arise then in the Sweco Turbo Screen. Consequently (see the teaching of EP-A-0915667), the process of EP-A-0574983 is not suitable for finely ground aspartame.

Preferably, the APM so-obtained according to the invention, and having a d₁₀ value of at least 3 µm and a d₅₀ value in the range of from 15 to 65 µm, is prepared by controlling the mechanical size reduction so as to obtain a first product consisting of particles, which in the main are smaller than 80 µm.

More preferably, the APM according to the invention is classified to having a d₁₀ value of at least 4 µm and a d₅₀ value in the range of from 16 to 45 µm.

The invention, thus, leads to novel APM fine-grade products. The α-L-aspartyl-L-phenylalanine methyl ester, which has been produced by crystallisation under agitated conditions, granulation and subsequent mechanical size reduction of the particles formed, and classified to having a particle size that is in the main smaller than 100 µm, with a d₁₀ value of at least 3 µm, a d₅₀ value in the range of from 15 to 65 µm, is a novel product, and generally will have a free bulk density of at least 270 kg/m³.

Preferably, the α-L-aspartyl-L-phenylalanine methyl ester according to the invention has a particle size that is in the main smaller than 80 µm, has a d₁₀ value of at least 3 µm, a d₅₀ value in the range of from 15 to 65 µm, and a free bulk density of at least 270 kg/m³.

Such novel APM products according to the invention also can be characterized and distinguished from the products of the prior art by their graphical representation in a so-called RRSB grid. The term RRSB grid (the abbreviation referring to the names of the scientists involved in development of such representation of particle size distribution, namely Rosin, Rammler, Sperling and Bennett), as meant herein, means a graphical representation (X-axis logarithmic for particle size; Y-axis double logarithmic for weight %), and scales, both for the percentual distribution (0 to 100%) of particle sizes on the Y-axis and for the particle sizes, e.g. 0 to 100 µm, on the X-axis. Generally, for products obtained by mere particle size reduction, e.g. by milling, the particle size distribution of the products obtained is represented by a straight line in an RRSB grid. Products, which subsequent to such grinding or milling step, undergo further classification, no longer will be represented linearly in an RRSB graph (i.e. the graphical representation of the particle size distribution in an RRSB grid). RRSB grids are being described in literature, for instance, in Mechanische Verfahrenstechnik I, VEB Deutscher Verlag für Grundstoffindustrie, Leipzig, 1977, page 27-28.

The present invention therefore also relates to novel APM products which are being characterized in that RRSB graphs of the APM products according to the invention show a substantially steeper slope in the range between d₂₅ and d₉₀ than in the range between d₁₀ and d₂₀.

The APM that can be used as starting material in the method according to the invention must be obtained through crystallisation from an aqueous medium with forced convection. Methods for crystallisation of APM with forced convection are known as such to a person skilled in the art; in the context of the present invention there are no exceptions to these methods, providing that they are carried out in an aqueous medium. The APM can also be obtained through neutralisation crystallisation from corresponding salts, such as APM.HCl salt. "'Aqueous medium" is in this context understood to be water or water that contains a limited concentration, up to for example at most 25 wt.%, of a lower alcohol (C₁-C₃). The forced convection can for example be realised via circulation of (part or all of) the solution used for crystallisation, or by keeping the solution used for crystallisation in motion by stirring or otherwise. The crystallisation can be effected for example via direct or indirect cooling, or by removing the aqueous solvent through evaporation. Of course, APM that has been obtained via crystallisation without forced convection (i.e. obtained via so-called static crystallisation) can also be converted into APM that is usable in the context of the present method, via re-crystallisation with forced convection.

The solid APM that is formed in the crystallisation can then be separated from the remaining aqueous medium in any manner known to a person skilled in the art and then be dried and granulated, also in a known manner, and then be reduced in size, for example by grinding. Examples of drying methods that are suitable for use are: fluid-bed drying, microwave drying, vacuum drying, etc. Examples of granulation methods that are suitable for use are wet granulation, compacting granulation, etc. The drying and granulation may also be combined in one process, for example by using a high-speed paddle dryer (HSPD).

The invention will hereinafter be elucidated with reference to some examples and comparative experiments, without being in any way whatsoever limited thereby.

Where relevant, use was made of the following products, techniques, methods and equipment:

Free bulk density (or f.b.d.) is determined according to ASTM D1895-89 (1990).

Particle size distribution is determined by laser diffraction method, using Sympatec Helos KFS laser diffraction R3 equipment, with optics suitable for the particle size range from 0,9 to 175 µm. Measurements were done in a 56 cm³ cuvette, under stirred conditions (magnetic stirrer, double cross type; diameter 14 mm; at 800 r.p.m.). Small amounts (spatula tip) of APM were introduced into 50 cm³ of hexane in the cuvette, and measurement was carried out for about 20 minutes until a stable p.s.d. was found (by Fraunhofer model calculations based on the diffraction pattern).

*ff*_{*c*} Values were determined using a Jenike shear tester according to the methods described by J. Schwedes, in Powder Handling & Processing, Vol.12(4), October/December 2000, pages 337-354, especially at page 341. The tests after 2 weeks of storage were performed under a pressure of 3275 N/m². To prevent moisture uptake the Jenike shear cell with the product was stored in a plastic bag, in a conditioned room at 25°C and a relative humidity of 75%.

Two granular products of APM, produced by agitated crystallisation and subsequent solid/liquid separation, drying and granulation, and finally separation of particle size fractions, namely for product A between about 250 and about 700 µm, and for product B between about 250 and about 1400 µm, were used as starting material.

Particle size reduction of granular APM was done both on an ACM10 and on an ACM30 mill of Hosokawa Micron GmbH, Köln, Germany. These types of milling equipment are different as to production capacity, but not as to operating principles. These mills contain a classifying part to adjust the upper limit of the particle size distribution, and were operated respectively at 3000 r.p.m. and at 2500 r.p.m. The mills were operated at their standard operational load in such way, i.e. by adjusting the air flow (using conditioned air of ambient temperature and a relative humidity of 40%), that the APM particle size of the granular products was reduced so as to give a fine grade product with 100 wt.% of the particles smaller than 80 µm. The air flow in the ACM10 was set at 15 m³/min, and in the ACM30 at 14 m³/min.

Removing fine particles therefrom was done either by using a MikroClassifier CC 30 (Hosokawa Micron GmbH) or an ATP 50 classifier (Hosokawa Alpine, Augsburg, Germany). Both classifiers are deflector wheel classifiers. The CC 30 classifier was operated at a tip speed of 70 m/s, and an air flow of 15 m³/min (primary airflow; counterflow), respectively 1,8 m³/min (secondary flow). The ATP 50 classifier was operated at a tip speed of 40 m/s, and an average air flow of 65 m³/h (counterflow).

The results of these experiments are shown in Table 1, which also includes the p.s.d. data (respectively d₁₀, d₅₀ and d₉₀), f.b.d. values and *ff*_{*c*} values (direct and after 2 weeks) for the (comparative) products before the removing of fines.

**Table 1**

| Comp.Ex. or Example | Mill + coarse classifier | Deflector wheel classifier | Product properties determined | | | | | |
|---|---|---|---|---|---|---|---|---|
| | | | d₁₀ (µm) | d₅₀ (µm) | d₉₀ (µm) | f.b.d. kg/m³ | *ff*_{*c*} direct | *ff*_{*c*} 2 *wks* |
| | | | | | | | | |
| Comp. A | ACM10 | no | 1,2 | 5,2 | 17,0 | 188 | 1,5 | 0,6 |
| | | | | | | | | |
| Comp. B | ACM30 | no | 2,1 | 10,5 | 41,8 | 187 | 1,5 | 0,6 |
| | | | | | | | | |
| Ex.1 | ACM10 | CC30 | 3,7 | 18,9 | 42,1 | 307 | 2,9 | 1,9 |
| | | | | | | | | |
| Ex.2 | ACM30 | ATP50 | 7,0 | 18,9 | 34,5 | 338 | 2,4 | 1,0 |
| | | | | | | | | |
| Ex.3 | ACM30 | ATP50 | 9,6 | 22,8 | 45,0 | 406 | 3,6 | 1,9 |

This table shows that the products of Examples 1, 2 and 3 have less caking than in the products of Comparative Examples A and B. The effect on balling was tested by mild mixing small amounts of these APM products with cocoa. For the products of Examples 1, 2 and 3 presence of APM could not be observed by bare eye, whereas in the products of Comparative Examples A and B many white spots were observed due to balling of the APM.

## Claims

1. Method for producing an improved fine grade of α-L-aspartyl-L-phenylalanine methyl ester having a particle size that is in the main smaller than 100 µm from α-L-aspartyi-L-phenylalanine methyl ester which has been produced by crystallisation under agitated conditions, granulation and subsequent mechanical size reduction of the particles formed, **characterized in that** the mechanical size reduction of the α-L-aspartyl-L-phenylalanine methyl ester is carried out so as to obtain a first product consisting of particles, which in the main are smaller than 100 µm, from which in a further step the fraction of finest particles is removed to a large extent by dry classifying and α-L-aspartyl-L-phenylalanine methyl ester is obtained having a d₁₀ value of at least 3 µm and a d₅₀ value in the range of from 15 to 65 µm, the d-values as determined by laser diffraction particle size measurement.

2. Method according to claim 1, **characterized in that** the mechanical size reduction of α-L-aspartyl-L-phenylalanine methyl ester is carried out so as to obtain in the mechanical reduction step a first product consisting of particles, which in the main are smaller than 80 µm.

3. Method according to claim 1 or 2, **characterized in that** α-L-aspartyl-L-phenylalanine methyl ester is obtained having a d₁₀ value of at least 4 µm and a d₅₀ value in the range of from 16 to 45 µm.

4. α-L-Aspartyl-L-phenylalanine methyl ester having a particle size that is in the main smaller than 100 µm, having a d₁₀ value of at least 3 µm, a d₅₀ value in the range of from 15 to 65 µm, and a free bulk density of at least 270 kg/m³.

5. α-L-Aspartyl-L-phenylalanine methyl ester having a particle size that is in the main smaller than 80 µm, having a d₁₀ value of at least 3 µm, a d₅₀ value in the range of from 15 to 65 µm, and a free bulk density of at least 270 kg/m³.

6. α-L-Aspartyl-L-phenylalanine methyl ester, **characterized in that** the particle size distribution of said α-L-aspartyl-L-phenylalanine methyl ester when represented graphically in a so-called Rosin-Rammler-Sperling-Bennett (RRSB) grid shows a substantially steeper slope in the range between d₂₅ and d₉₀ than in the range between d₁₀ and d₂₀.
